# EUROPÄISCHE PATENTSCHRIFT

(11) **EP 2 492 832 B1**
(45) Veröffentlichungstag und Bekanntmachung des Hinweises auf die Patenterteilung: **18.08.2021**
(21) Anmeldenummer: 11155405.1
(22) Anmeldetag: 22.02.2011
(51) Int. Cl.: G16H 40/67

(54) **OPTIMIERUNG EINER AUF EINEM CLIENT-SERVER-SYSTEM IMPLEMENTIERTEN SOFTWAREAPPLIKATION**
OPTIMISATION OF A SOFTWARE APPLICATION IMPLEMENTED ON A CLIENT SERVER SYSTEM
OPTIMISATION D'UNE APPLICATION LOGICIELLE IMPLÉMENTÉE DANS UN SYSTÈME CLIENT-SERVEUR

(43) Veröffentlichungstag der Anmeldung: 29.08.2012
(73) Patentinhaber: Siemens Healthcare GmbH, 91052 Erlangen (DE)
(72) Erfinder: Dorn, Karlheinz, 90562, Kalchreuth (DE); Dominick, Lutz, 91330, Eggolsheim (DE); Ukis, Vladyslav, 90491, Nürnberg (DE)

(56) Entgegenhaltungen:
- US-A1- 2002 174 293
- US-A1- 2003 090 515
- US-A1- 2010 153 313
- None

## Beschreibung

Die Erfindung bezieht sich auf ein Verfahren zur automatischen Optimierung einer auf einem Client-Server-System implementierten Software-Applikation. Die Erfindung bezieht sich des Weiteren auf eine Einrichtung zur Durchführung des Verfahrens. Das Verfahren und die zugehörige Einrichtung dienen insbesondere zur automatischen Optimierung einer medizintechnischen Software-Applikation für die Anzeige und Befundung von digitalen medizinischen Bilddaten.

Moderne medizinische (Software-)Applikationen, d.h. Anwendungsprogramme, verfügen regelmäßig über eine große Anzahl von Funktionen, die einem Nutzer erlauben, medizinische Bilddaten anzuzeigen, zu bearbeiten und zu befunden. Für jede Funktion, oder zumindest für jede zusammengehörige Gruppe von Funktionen ist in der Regel eine bestimmte Nutzeroberfläche (User Interface, kurz: UI) mit zugehörigen Bedienelementen wie z.B. Buttons, Textfeldern, Tasten- und/oder Mauskombinationen, Layouts etc. definiert, die eine möglichst optimale Nutzbarkeit der Funktion oder Funktionsgruppe ermöglichen sollen. Im Rahmen einer Befundung werden hierbei meist mehrere Funktionen oder Funktionsgruppen nacheinander benutzt, die den Nutzer, d.h. einen die Applikation bedienenden Arzt, zu einem Befundungsergebnis führen. Ein Maß für die Qualität der Nutzbarkeit der Applikation stellt die "Performance" der Applikation dar, die sich wiederum in der Anzahl der medizinischen Bilddatensätze äußert, die von einem Nutzer im Durchschnitt pro Zeiteinheit mittels der Applikation befundet werden.

Die Performance einer medizintechnischen Applikation hängt in der Praxis von einer Vielzahl von Faktoren ab, unter anderem auch von der Wechselwirkung der (Software-)Applikation mit den hardwaretechnischen Gegebenheiten des Client-Server-Systems, auf dem die Applikation implementiert ist, sowie von der bevorzugten Arbeitsweise und den Fähigkeiten der individuellen Nutzer, die mit der Applikation arbeiten.

Viele dieser Faktoren hängen somit von den spezifischen Betriebsbedingungen einer medizinischen Applikation am individuellen Einsatzort ab, und können deshalb bei der Entwicklung der Applikation a priori nur eingeschränkt vorausgesehen und berücksichtigt werden. Die im Betrieb einer medizinischen Applikation tatsächlich erreichte Performance ist daher regelmäßig vergleichsweise weit von dem erzielbaren Optimum entfernt. Angesichts der Komplexität einer modernen medizinischen Applikation ist andererseits eine individuelle Anpassung einer solchen Applikation an die jeweiligen Einsatzbedingungen aus Gründen des damit verbundenen Aufwands häufig nicht oder nur ansatzweise möglich.

Die US 2003 / 0 090 515 A1 offenbart eine adaptive Benutzeroberfläche und ein darauf basierendes Verfahren zur Vereinfachung der Verwendung neuer elektronischer Geräte. Dabei wird die Benutzeroberfläche auf der Grundlage des Nutzungsverlaufs angepasst. Die adaptive Benutzeroberfläche überwacht Eingabebefehle für die Benutzeroberfläche und verfolgt Nutzungsstatistiken, um zu bestimmen, ob Funktionen zur Benutzeroberfläche hinzugefügt oder daraus entfernt werden sollen. Dabei werden, je versierter der Benutzer wird, automatisch weitere Funktionalitäten hinzugefügt. Umgekehrt werden selten verwendete Funktionalitäten automatisch entfernt. Durch Verfolgen der Benutzeraktionen kann die Benutzeroberfläche genauer an die tatsächlichen Nutzungsgewohnheiten jedes Benutzers angepasst werden.

Der Erfindung liegt vor diesem Hintergrund die Aufgabe zu Grunde, ein effektives und einfach umsetzbares Verfahren zur Optimierung einer auf einem Client-Server-System implementierten (insbesondere medizinischen) Softwareapplikation hinsichtlich der im Betrieb der Applikation erzielten Performance anzugeben. Der Erfindung liegt weiterhin die Aufgabe zugrunde, eine zur automatischen Durchführung des Verfahrens geeignete Einrichtung anzugeben.

Bezüglich des Verfahrens wird diese Aufgabe erfindungsgemäß gelöst durch die Merkmale des Anspruchs 1. Bezüglich der zur Durchführung des Verfahrens geeigneten Einrichtung wird die Aufgabe erfindungsgemäß gelöst durch die Merkmale des Anspruchs 4. Vorteilhafte Ausführungen und Weiterentwicklungen der Erfindung sind durch die Unteransprüche gegeben.

Hinsichtlich der zu optimierenden Softwareapplikation wird unterschieden zwischen einem Applikations-Frontendmodul, das auf dem oder jedem Client des Client-Server-Systems implementiert ist, und einem Applikations-Backendmodul, das auf einem oder mehreren Server(n) des Client-Server-Systems implementiert ist. Die erfindungsgemäße Einrichtung umfasst ein clientseitig implementiertes Überwachungs-Frontendmodul sowie ein serverseitig implementiertes Überwachungs-Backendmodul.

Mittels des Überwachungs-Frontendmoduls werden dabei verfahrensgemäß während der Ausführung der Applikation fortlaufend Daten über die Nutzung und den Betriebszustand des Applikations-Frontendmoduls erhoben. Diese Nutzungs- und Zustandsdaten werden von dem Überwachungs-Frontendmodul an das Überwachungs-Backendmodul übermittelt, von welchem die Nutzungs- und Zustandsdaten in einer serverseitig angelegten (Nutzungs-)Datenbank hinterlegt werden.

Die erfindungsgemäße Einrichtung umfasst des Weiteren ein Analysemodul, das insbesondere ebenfalls serverseitig implementiert ist. Mittels dieses Analysemoduls werden die in der Datenbank hinterlegten Nutzungs- und Zustandsdaten automatisch analysiert, wobei das Analysemodul verfahrensgemäß auf Basis des Analyseergebnisses automatisch Voreinstellungen der Applikation zur Verbesserung der Applikationsperformance ändert.

Der Begriff "Voreinstellungen" umfasst hierbei allgemein alle Daten, die Einfluss auf die Arbeitsweise der Applikation haben, und die änderbar sind, ohne die Applikation neu kompilieren zu müssen. Zu den Voreinstellungen gehören dabei insbesondere Konfigurationsdaten, die den Funktionsumfang oder den Ablauf der Applikation beeinflussen, die das Erscheinungsbild einer Nutzeroberfläche der Applikation bestimmen oder die Quell- und Zielpfade im Dateisystem des Client-Server-Systems definieren. Zu den Voreinstellungen gehören des Weiteren Verzeichnisse für Vorlagen und Musterdateien, auf die die Applikation zugreift.

Bei einer Applikation zur Befundung von digitalen medizinischen Bilddaten umfassen die Voreinstellungen insbesondere einen sogenannten "Layoutspeicher" (Layout Repository), in dem sogenannte Layouts gespeichert sind, auf die die Nutzeroberfläche der Applikation wahlweise zugreifen kann. Als "Layout" wird hierbei ein Datensatz bezeichnet, der die Anzahl und Anordnung der auf der Nutzeroberfläche darzustellenden Bilder festlegt.

Die Voreinstellungen der medizinischen Applikation umfassen des Weiteren zweckmäßigerweise einen "Applikationsvorlagenspeicher" (App Template Repository), in dem sogenannte "Applikationsvorlagen" (App Templates) zur Auswahl vorgehalten werden. Jede Applikationsvorlage definiert hierbei einen Funktionsumfang, mit dem das Applikations-Frontendmodul auf einem Client gestartet werden kann, insbesondere eine Anzahl von gegebenenfalls zusätzlich zu den Grundfunktionen der Applikation zuzuladenden Plug-Ins, eine Auswahl der Bedienelemente für die Nutzeroberfläche und - soweit es sich um graphische Bedienelemente handelt - Vorgaben zu deren Anordnung auf der Nutzeroberfläche, etc.

Die Voreinstellungen der medizinischen Applikation umfassen darüber hinaus in zweckmäßiger Ausführung einen "Nutzereinstellungsspeicher" (User Settings Repository), in dem für jeden Client-Nutzer ein "Nutzerprofil", d.h. ein Set von individuellen Nutzereinstellungen gespeichert ist. Jedes Nutzerprofil umfasst dabei insbesondere Verweise auf die von dem Nutzer für bestimmte Anwendungsfälle bevorzugten Layouts, Verweise auf die dem Nutzer zur Verfügung stehenden Applikationsvorlagen sowie ferner Angaben zur Identität des jeweiligen Nutzers, insbesondere Name, Login-Identifikation und Passwort.

Optional werden durch das Überwachungs-Frontendmodul als Nutzungs- und Zustandsdaten insbesondere Angaben zu
- der Identität eines Client-Nutzers,
- der oder jeden von dem Client-Nutzer aufgerufenen Applikation,
- den Eigenschaften des Layouts der Applikation,
- dem Zustand einer Nutzeroberfläche der Applikation, insbesondere Angaben zu einem der Nutzeroberfläche ggf. zugrundeliegenden Applikationstemplate,
- den von der Applikation geladenen Daten,
- den an die Applikation gerichteten Nutzerbefehlen, sowie
- dem Interaktionsstartzeitpunkt
erfasst.

In einer Variante werden die in der Nutzungsdatenbank hinterlegten Nutzungs- und Zustandsdaten durch das Analysemodul automatisch auf sich wiederholende Befehlsfolgen eines Client-Nutzers durchsucht. Verfahrensgemäß wird durch das Analysemodul dabei zu einer solchen Befehlsfolge ein "Nutzungsschema" erzeugt und in den Voreinstellungen der Applikation hinterlegt.

Als "Befehl" wird hierbei jegliche Interaktion verstanden, die ein Client-Nutzer mit der Applikation eingeht. Ein solcher Befehl kann formal insbesondere in dem Anklicken eines Buttons oder der Auswahl eines Menüpunktes einer grafischen Nutzeroberfläche, der Markierung eines Flächenbereiches auf der Nutzeroberfläche oder in dem Drücken einer bestimmten Taste oder Tastenkombination bestehen, und beispielsweise das Laden von bestimmten Daten aus einem Speicher, das Anzeigen der geladenen Daten in einem bestimmten Bereich einer Nutzeroberfläche, die Anwendung von bestimmten Funktionen der Applikation, insbesondere Bildbearbeitungsalgorithmen, auf die geladenen Daten und/oder das Speichern der (gegebenenfalls veränderte) Daten zum Inhalt haben.

Als "Nutzungsschema" wird allgemein ein Programm verstanden, das im Rahmen der Applikation die jeweilige Befehlsfolge automatisch ausführt. Bei dem "Nutzungsschema" handelt es sich insbesondere um ein sogenanntes "Makro" oder einem "Arbeitsablauf" (Workflow).

Stellt das Analysemodul beispielsweise fest, dass ein Client-Nutzer wiederholt nach dem Laden eines bestimmten Typs von Bilddatensätzen einen Bildsynthese (Volume Rendering, kurz: VR)-Algorithmus oder eine Maximumintensitätsprojektion (MIP) auf die geladenen Bilddaten anwendet, erzeugt das Analysemodul in der vorstehend beschriebenen Ausführungsvariante des Verfahrens automatisch ein Nutzungsschema, im Rahmen dessen der Bildsynthesealgorithmus bzw. die Maximumintensitätsprojektion automatisch durchgeführt wird, sobald der Client-Nutzer einen Bilddatensatz des entsprechenden Typs lädt.

Grundsätzlich kann vorgesehen sein, dass das Analysemodul bereits bei der ersten festgestellten Wiederholung einer Befehlsfolge ein entsprechendes Nutzungsschema erzeugt. Um die Anzahl der erzeugten Nutzungsschemata auf ein für einen Client-Nutzer überschaubares Maß einzugrenzen, sind dem Analysemodul für die Erzeugung eines Nutzungsschemas zu einer sich wiederholenden Befehlsfolge aber vorzugsweise weitere einschränkende Bedingungen vorgegeben.

So erzeugt das Analysemodul in einer zweckmäßigen Ausführungsform ein Nutzungsschema lediglich zu einer Befehlsfolge, die sich in den hinterlegten Nutzungs- und Zustandsdaten mehrfach, beispielsweise mehr als zehn Mal wiederholt. Die erforderliche Anzahl von Wiederholungen einer Befehlsfolge kann weiterhin zusätzlich an einen Zeitraum gekoppelt sein, so dass das Analysemodul beispielsweise ein Nutzungsschema nur dann erzeugt, wenn eine entsprechende Befehlsfolge sich innerhalb eines bestimmten Zeitraumes mehr als x-mal (x=3,4,5,...) wiederholt. In zusätzlicher oder alternativer Ausführung ist als weitere beschränkte Bedingung vorgesehen, das ein Nutzungsschema nur zu Befehlsfolgen erzeugt wird, die eine Mindestanzahl von n (mit n = 3,4,5,...) Befehlen umfassen.

Grundsätzlich kann weiterhin vorgesehen sein, dass das Analysemodul das oder jedes Nutzungsschema derart hinterlegt, dass das Nutzungsschema ohne weiteres Zutun des Client-Nutzers ausgeführt wird, sobald der Client-Nutzer den ersten Befehl der zugehörigen Befehlsfolge auslöst. Vorzugsweise wird im Rahmen des oder jedes Nutzungsschemas aber eine Rückfrage erzeugt, in der der Client-Nutzer - zumindest bei der erstmaligen Ausführung des Nutzungsschemas - aufgefordert wird, die automatische Ausführung des Nutzungsschemas zu bestätigen oder abzulehnen.

Die erzeugten Nutzungsschemata können durch das Analysemodul global, d.h. für alle Clients und Client-Nutzer zugänglich hinterlegt werden. Abweichend hiervon ist optional vorgesehen, dass die Nutzungsschemata nutzerspezifisch ermittelt und/oder hinterlegt werden. Zusätzlich oder alternativ hierzu ist gemäß einer Weiterentwicklung vorgesehen, dass die Nutzungsschemata segmentspezifisch ermittelt und hinterlegt werden. Das Analysemodul bestimmt hierbei, an welchem Segment einer Nutzeroberfläche der Applikation wiederholt gleiche Befehlsfolgen vorgenommen werden, und erzeugt entsprechende Nutzungsschemata, die ausschließlich auf das zugehörige Segment angewendet werden. Stellt das Analysemodul beispielsweise fest, dass ein bestimmter Datenbearbeitungsalgorithmus von einem oder mehreren Client-Nutzern regelmäßig auf Daten eines bestimmten Segments angewendet werden, erzeugt es ein Nutzungsschema, das diesen Bildbearbeitungsalgorithmus automatisch auf die in diesem zugehörigen Segment angezeigten Daten anwendet.

Als "Segment" wird hierbei allgemein ein Bereich einer Nutzeroberfläche bezeichnet, der durch Nutzerinteraktion reversibel markiert oder aktiviert werden kann, wobei ein aktiviertes bzw. markiertes Segment einen Vordergrund der Nutzeroberfläche bildet. Im Rahmen einer grafischen Nutzeroberfläche kann ein solches Segment beispielsweise als "Fenster" oder "Registerkarte" ausgebildet sein. Im Falle einer Applikation zur Befundung von digitalen Bilddaten ist ein solches Segment insbesondere durch ein Anzeigefeld für einen medizinischen Bilddatensatz gebildet.

In einer weiteren Variante ist vorgesehen, dass durch das Analysemodul anhand der Analyse der hinterlegten Nutzungs- und Zustandsdaten dasjenige Segment einer mehrere Segmente umfassenden Nutzeroberfläche ermittelt wird, mit dem ein Client-Nutzer nach dem Starten des Applikations-Frontendmoduls oder bei einem mittels des Applikations-Frontendmoduls durchgeführten Arbeitsablaufs (Workflow) mit der höchsten Wahrscheinlichkeit zuerst interagiert. Verfahrensgemäß ändert das Analysemodul die Voreinstellungen der Applikation dabei derart, dass das ermittelte Segment beim Starten des Applikationsfrontendmoduls bzw. zum Bearbeitungsbeginn des Arbeitsablaufs automatisch in den Vordergrund gesetzt wird.

Als "Vordergrund" wird derjenige Teil einer Nutzeroberfläche bezeichnet, mit dem der Client-Nutzer unmittelbar interagieren kann. In Abgrenzung zu dem Vordergrund der Nutzeroberfläche bilden nicht unmittelbar beeinflussbare oder verborgene Bestandteile der Nutzeroberfläche, insbesondere nicht-aktive Fenster oder verborgene Registerkarten, einen "Hintergrund" der Nutzeroberfläche.

Mit dem Begriff "Arbeitsablauf" (Workflow) wird allgemein eine im Rahmen der Applikation definierte und implementierte, mehrschrittige Tätigkeit bezeichnet. Bei einer Applikation zur Befundung medizinischer Bilddaten können als Arbeitsabläufe beispielsweise die Befundung eines Mammographiedatensatzes oder die Befundung eins MR-Tomographiedatensatzes des Kopfes implementiert sein.

In einer weiteren Ausführungsform ist vorgesehen, dass durch das Analysemodul anhand der Analyse der hinterlegten Nutzungs- und Zustandsdaten die (Betätigungs-)Häufigkeit bestimmt wird, mit welchem graphische Bedienelemente einer Nutzeroberfläche der Applikation jeweils betätigt werden. Das Analysemodul ändert hierbei verfahrensgemäß die Voreinstellungen der Applikation in Abhängigkeit des Analyseergebnisses derart, dass die Bedienelemente in einer von der Betätigungshäufigkeit abhängigen Ordnung auf der Nutzeroberfläche angeordnet werden. Insbesondere werden die Bedienelemente zweckmäßigerweise in der Reihenfolge ihrer Betätigungshäufigkeit angeordnet, im Falle von Menüpunkten eines üblichen "Drop-Down-Menüs" beispielsweise mit abnehmender Betätigungshäufigkeit von oben nach unten, oder im Falle einer Icon- oder Button-Reihe beispielsweise mit abnehmender Betätigungshäufigkeit von links nach rechts. Mit dieser Verfahrensvariante wird der Vorteil erzielt, dass häufig benutzte Bedienelemente nach der Umordnung für den Client-Nutzer leichter und schneller zugänglich sind.

In einer zweckmäßigen Weiterbildung ermittelt das Analysemodul anhand der festgestellten Betätigungshäufigkeit diejenigen Bedienelemente, die von einem Client-Nutzer nicht oder nur selten verwendet werden. Das Analysemodul ändert hierbei die Voreinstellungen der Applikation derart, dass die nicht oder selten verwendeten Bedienelemente und die zugehörigen Programmroutinen (Tools) der Applikation bei einem Neustart durch den betreffenden Client-Nutzer nicht mehr automatisch geladen werden, wodurch der Ressourcenbedarf der Applikation verringert wird.

Gemäß einer weiteren Variante ist vorgesehen, dass durch das Analysemodul die (Zugriffs-) Häufigkeit bestimmt wird, mit der mittels der Applikation auf bestimmte Datensätze zugegriffen wird. Das Analysemodul weist hierbei den überprüften Datensätzen einen jeweils nach Maßgabe der ermittelten Zugriffshäufigkeit bestimmten Speicherort innerhalb des Dateisystems des Client-Server-Systems zu. Insbesondere veranlasst das Analysemodul hierbei, dass Datensätze mit hoher Zugriffshäufigkeit in einem lokalen Speicher oder einem anderen Speicher mit kurzer Zugriffszeit vorgehalten werden, während Datensätze mit demgegenüber geringer Zugriffshäufigkeit in einem Archivspeicher mit längerer Zugriffszeit abgelegt werden. Durch diese Verfahrensvariante wird der Vorteil erzielt, dass häufig verwendete Datensätze für einen Client-Nutzer schnell zur Verfügung stehen, während andererseits lokale Speicherbereiche von Datensätzen mit geringer Zugriffshäufigkeit automatisch bereinigt werden, so dass einer Überlastung dieser Speicherbereiche entgegengewirkt wird.

In einer weiteren Variante wird durch das Analysemodul die (Aufruf-)Häufigkeit bestimmt, mit der die oder jede Applikation aufgerufen wird. Das Analysemodul ändert hierbei, wenn die ermittelte Aufrufhäufigkeit einer Applikation einen vorgegebenen Schwellwert überschreitet, die Voreinstellungen dieser Applikation derart ab, dass das zugehörige Applikations-Backendmodul bereits unabhängig von dem oder jedem Frontendmodul dieser Applikation gestartet und betrieben wird. In einer verfeinerten Ausführungsform dieser Erfindungsvariante wird die Aufrufhäufigkeit für die oder jede Applikation hierbei zeitaufgelöst bestimmt. Das Analysemodul bestimmt dabei insbesondere, zu welcher Tageszeit eine bestimmte Applikation besonders häufig durch Client-Nutzer gestartet wird. In diesem Fall startet das Analysemodul das Applikations-Backendmodul mit einem vorgegebenen Vorlauf vor den ermittelten Stoßzeiten(d.h. den Zeiten hoher Aufrufwahrscheinlichkeit). Der client-unabhängige Start des Applikations-Backendmoduls hat hierbei den Vorteil, dass dieses Applikations-Backendmodul in der überwiegenden Anzahl der Fälle bereits läuft, wenn ein Client-Nutzer die Applikation clientseitig aufruft. Dies hat den Vorteil, dass die Applikation in der Wahrnehmung des Client-Nutzers besonders schnell "hochfährt", dass also der Zeitabstand zwischen dem clientseitigen Aufruf der Applikation und der Arbeitsbereitschaft der Applikation besonders kurz ist.

In einer weiteren Variante ist vorgesehen, dass durch das Analysemodul der Verbrauch der Applikation an Ressourcen des Client-Serversystems bestimmt wird. Das Analysemodul verändert hierbei die Voreinstellungen der Applikation - insbesondere durch entsprechende Konfiguration eines Applikationsstarters - derart, dass die Applikation bei einem Aufruf durch einen Client-Nutzer nur dann gestartet wird, wenn an dem Client-Server-System genügend freie Ressourcen zur Verfügung stehen. Alternativ kann vorgesehen sein, dass die Applikation im Falle von nicht ausreichenden Ressourcen nur mit beschränktem Funktionsumfang, und entsprechend verringertem Ressourcenbedarf gestartet wird.

Die "Ressourcen" des Client-Server-Systems umfassen hierbei insbesondere die verfügbaren CPU-Leistung und den freien Arbeitsspeicherplatz sowie die freie Datenübertragungskapazität der benötigten Netzwerk- und Datenbuskomponenten des Client-Server-Systems.

Durch diese Erfindungsvariante wird insbesondere der Vorteil erzielt, dass eine Überlastung des Client-Server-Systems, die zu einer Verlangsamung aller bereits laufenden Applikationen und Prozesse führen würde, vermieden wird.

Um die Leistung des Client-Server-Systems durch den Ressourcenbedarf des Analysemoduls so wenig wie möglich zu belasten, ist vorzugsweise vorgesehen, dass das Analysemodul die in der Nutzungsdatenbank hinterlegten Nutzungs- und Zustandsdaten diskontinuierlich in regelmäßigen Zeitabständen, insbesondere einmal pro Tag analysiert und auch die entsprechenden Änderungen an den Voreinstellungen der oder jeder Applikation diskontinuierlich in entsprechenden Zeitabständen vornimmt. Das Analysemodul wird hierbei vorzugsweise in Zeiten mit besonders geringer Ressourcenauslastung tätig, insbesondere während der Nacht.

Ebenfalls um den Ablauf der oder jeder Applikation möglichst wenig zu stören, ist in zusätzlicher oder alternativer Ausführung vorzugsweise vorgesehen, dass das oder jedes Applikations-Frontendmodul die von ihm erhobenen Nutzungs- und Zustandsdaten asynchron, d.h. ohne Blockierung anderer Prozesse, und somit im Hintergrund der Applikation an das Überwachungs-Backendmodul übermittelt.

In weiteren Varianten ermittelt das Analysemodul anhand der Nutzungs- und Zustandsdaten Änderungen, die ein Client-Nutzer an dem Layout oder dem Zustand einer Nutzeroberfläche der Applikation vornimmt. Das Analysemodul erstellt in diesem Fall neue Layouts bzw. Applikationsvorlagen, die die von dem Client-Nutzer vorgenommenen Änderungen reflektieren. Diese Erfindungsvariante hat den Vorteil, dass die von einem Client-Nutzer vorgenommenen Änderungen an dem Layout oder dem Zustand der Nutzeroberfläche zu einem späteren Zeitpunkt automatisch wieder hergestellt sowie anderen Client-Nutzern zugängig gemacht werden können.

Nachfolgend wird ein Ausführungsbeispiel anhand einer Zeichnung näher erläutert. Darin zeigen:
- FIG 1: in einem schematischen Blockschaltbild ein Client-Server-System mit zwei darauf implementierten, medizintechnischen Applikationen sowie mit einer Einrichtung zur automatischen Optimierung dieser Applikationen, und
- FIG 2: in einem schematischen Ablaufdiagramm ein durch die Einrichtung gemäß Fig. 1 durchgeführtes Verfahren zur Optimierung der Applikationen.

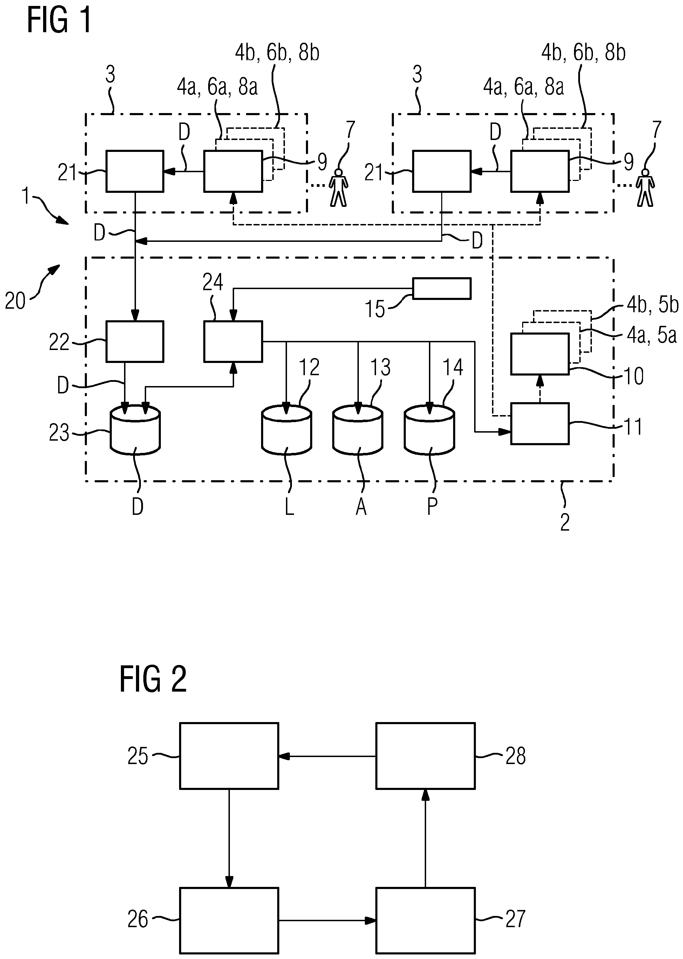

FIG 1 zeigt in grober schematischer Vereinfachung ein Client-Server-System 1, das in einer medizinischen Einrichtung, beispielsweise einer Radiologieabteilung einer Klinik eingesetzt ist. Das Client-Server-System 1 umfasst einen zentralen Server 2 sowie - vereinfacht dargestellt - zwei Clients 3. Bei den Clients 3 handelt es sich beispielsweise um gewöhnliche Arbeitsplatzrechner, insbesondere Personal Computer. Die Clients 3 sind mit dem Server 2 über ein nicht näher dargestelltes Datenübertragungsnetzwerk, beispielsweise ein Local-Area-Network (LAN) datenübertragungstechnisch verbunden.

Auf dem Client-Server-System 1 sind beispielhaft zwei medizinische (Software-)Applikationen 4a und 4b implementiert. Bei der Applikation 4a handelt es sich beispielsweise um ein Programm zur Anzeige, Bearbeitung und Befundung von CT-Bilddaten (Computertomogrammen). Bei der Applikation 4b handelt es sich beispielsweise um ein Programm zur Anzeige, Bearbeitung und Befundung von digitalen Ultraschall-Bildern.

Jede der beiden Applikationen 4a und 4b umfasst ein auf dem Server 2 implementiertes (Applikations-)Backendmodul 5a bzw. 5b. Jede der beiden Applikationen 4a und 4b umfasst des Weiteren eine Anzahl von (Applikations-)Frontendmodulen 6a bzw. 6b, von denen für jede Applikation 4a und 4b jeweils eines auf jedem der beiden Clients 3 implementiert ist. Die Frontendmodule 6a und 6b enthalten hierbei jeweils zumindest einen Teil der sogenannten "Präsentationslogik" der jeweiligen Applikation 4a und 4b, über welche die Applikationen 4a und 4b mit einem jeweiligen (Client-Nutzer) 7 interagieren. Insbesondere umfassen die Frontendmodule 6a und 6b hierzu jeweils eine grafische Nutzeroberfläche 8a bzw. 8b.

Die Applikations-Backendmodule 5a und 5b enthalten zumindest den überwiegenden Teil der sogenannten "Business-Logik", d.h. der eigentlichen Dienst-Programme zur Bild- und Textbearbeitung sowie zur medizinischen Befundung der Bilddaten. Die Verteilung (Deployment) des Funktionsumfangs der einzelnen Applikationen 4a und 4b und der zugehörigen Softwareschichten auf den Server 2 und die Clients 3 kann aber unterschiedlich gelöst sein. Je nach dem Anteil der clientseitig angeordneten Softwareschichten können die Frontendmodule 6a und 6b dabei insbesondere als sogenannter "Fat Client", "Rich Client", "Smart Client", "Rich Thin Client" oder "Web Client" ausgebildet sein. Das Deployment der Applikation 4a kann hierbei auch verschieden von dem Deployment der Applikation 4b sein.

Der sogenannte "Life-Cycle" der Applikationen 4a und 4b, d.h. die mit dem Starten, Beenden und Suspendieren der jeweiligen Applikation 4a und 4b zusammenhängenden Vorgänge werden clientseitig gesteuert durch jeweils ein, jedem der beiden Clients 3 zugeordnetes (Laufzeitsteuerungs-)Frontendmodul 9 (App Frontend Runner), das die jeweiligen Frontendmodule 6a, 6b der Applikationen 4a bzw. 4b startet, stoppt oder suspendiert. Den Life-Cycle der Applikations-Backendmodule 5a und 5b steuert entsprechend ein serverseitig implementiertes (Laufzeitsteuerungs-)Backendmodul 10 (App Backend Runner). Die Module 9 und 10 werden wiederum gemeinsam angesteuert durch ein als Applikationsstarter 11 bezeichnetes Softwaremodul, das ebenfalls serverseitig implementiert ist.

Zur Ermittlung der - gegebenenfalls client- und/oder nutzerspezifisch vorgegebenen - Konfiguration der Applikationen 4a und 4b greifen die Laufzeitsteuerungs-Frontendmodule 9 und das Laufzeitsteuerungs-Backendmodul 10 auf ein Set von Voreinstellungen zu, die der Applikation 4a und 4b jeweils zugeordnet sind. Diese Voreinstellungen sind strukturiert hinterlegt in einem (Layout-)Verzeichnis 12, einem (Applikationsvorlagen-) Verzeichnis 13 sowie einem (Nutzereinstellungs)Verzeichnis 14.

Das Layout-Verzeichnis 12 enthält hierbei eine Anzahl von der jeweiligen Applikation 4a oder 4b zugeordneten Layouts L, die jeweils eine Anzahl von Segmenten für die Nutzeroberfläche 8a bzw. 8b eines Clients 3, sowie die Größe und Anordnung dieser Segmente vorgibt. Bei jedem Segment handelt es sich hierbei um ein Anzeigefeld, in dem ein medizinisches Bild darstellbar ist.
In dem Applikationsvorlagen-Verzeichnis 13 sind für jede Applikation 4a und 4b sogenannte Applikationsvorlagen A hinterlegt. Jede Applikationsvorlage A definiert hierbei für die zugehörige Applikation 4a oder 4b einen Funktionsumfang. So ist im Rahmen einer jeden Applikationsvorlage A insbesondere festgelegt, welche Bedienelemente auf der Nutzeroberfläche 8a bzw. 8b der jeweiligen Applikation 4a oder 4b dargestellt werden sollen, und wie diese Bedienelemente angeordnet werden sollen.

Das Nutzereinstellungsverzeichnis 14 umfasst für jeden Client-Nutzer 7 ein Nutzerprofil P, das unter anderem Angaben zu den von dem individuellen Client-Nutzer 7 für bestimmte Anwendungsfälle bevorzugten Layouts L sowie zu den dem Nutzer 7 zur Verfügung stehenden Applikationsvorlagen A enthält. Das Nutzerprofil P enthält hierbei insbesondere Verweise (links) auf die entsprechenden Layouts L im Layout-Verzeichnis 12 bzw. Applikationsvorlagen A im Applikationsvorlagen-Verzeichnis 13.

Auf dem Server 2 ist ferner ein (Daten-)Archivierungsmodul 15 implementiert, das periodisch eine Archivierung der in einem lokalen Speicher des Servers 2 vorgehaltenen Bilddaten in einem Archiv oder Langzeitspeicher durchführt.

Auf dem Client-Server-System 1 ist des Weiteren eine Einrichtung 20 zur Optimierung der Performance der beiden Applikationen 4a und 4b implementiert. Diese Einrichtung 20 umfasst eine Anzahl von Überwachungs-Frontendmodulen 21, von denen jeweils eines einem jedem der Clients 3 zugeordnet ist. Die Einrichtung 20 umfasst des Weiteren ein serverseitig implementiertes Überwachungs-Backendmodul 22, eine Nutzungsdatenbank 23 sowie ein Analysemodul 24.

Bei den vorstehend genannten Modulen 5a,5b,6a,6b,9,10,11, 12,13,14,15,21,22,23,und 24 handelt es sich um Softwaremodule.

Ausgangspunkt und Vorbedingung für das mittels der Einrichtung 20 durchgeführte Optimierungsverfahren ist gemäß FIG 2 der Normalbetrieb des Client-Server-Systems 1, in dem mindestens eine der beiden Applikationen 4a oder 4b von mindestens einem der Client-Nutzer 7 verwendet wird, so dass mindestens ein Frontendmodul 6a,6b sowie das Backendmodul 5a,5b dieser Applikation 4a,4b laufen.

Nach dem Starten der Applikation 4a oder 4b (Schritt 25 in FIG 2) sammelt das Überwachungs-Frontendmodul 21 durch Wechselwirkung mit dem Laufzeitsteuerungs-Frontendmodul 9 des jeweiligen Clients 3 fortlaufend Daten D über die Nutzung und den Zustand des oder jedes auf diesem Client 3 installierten Applikations-Frontendmoduls 6a,6b. Diese (Nutzungs- und Zustands-)Daten D umfassen insbesondere Angaben zu
- der Identität des an dem jeweiligen Client 3 arbeitenden (und hierfür "eingeloggten") Client-Nutzers 7,
- die auf dem jeweiligen Client 3 laufenden Applikationen 4a,4b,
- das aktuell verwendete Layout L der oder jeder Applikation 4a, 4b,
- den aktuellen Zustand der Nutzeroberfläche 8a bzw. 8b, insbesondere den Zustand der Buttons, Textfelder, Routinen sowie des Präsentations-Status der Nutzeroberfläche 8a,8b (als "Präsentations-Status" wird hierbei der Zustand der Nutzeroberfläche bezeichnet, in dem der Client-Nutzer 7 eine Studie hinterlässt; zum Beispiel wird die Studie in einem Layout mit 16 in einer 4 x 4-Matrix angeordneten Bildern hinterlassen, wobei der Client-Nutzer 7 beispielsweise in ein erstes Bild zwei Distanzlinien eingefügt hat, und wobei der Client-Nutzer 7 beispielsweise zu dem fünften und zehnten Bild jeweils einen medizinischen Befund notiert hat),
- die von der jeweiligen Applikation 4a und 4b geladenen Daten,
- den Arbeitsmodus der jeweiligen Applikation 4a,4b, falls die Applikation 4a,4b in mehreren Arbeitsmodi betreibbar ist (im Falle der Applikation 4a stehen beispielsweise ein 2D-Modus, ein 3D-Modus sowie ein Bildsynthese-Modus zur Auswahl) sowie,
- den Startzeitpunkt der Nutzerinteraktion.

Jedes der beiden Überwachungs-Frontendmodule 21 sendet die von ihm gesammelten Nutzungs- und Zustandsdaten D an das serverseitig implementierte Überwachungs-Backendmodul 22. Die Übertragung der Nutzungs- und Zustandsdaten D erfolgt hierbei asynchron als Hintergrundprozess (Background-Job), um die Performance des jeweiligen Clients 3 nicht zu beeinträchtigen. Die gesammelten und an den Server 2 gesendeten Nutzungs- und Zustandsdaten D werden von dem Überwachungs-Backendmodul 22 in strukturierter Form in die Nutzungsdatenbank 23 eingespeist. Das Sammeln der Nutzungs- und Zustandsdaten D, deren Übermittlung von dem jeweiligen Client 3 an den Server 2 sowie die Einspeisung dieser Daten in die Nutzungsdatenbank 23 sind in FIG 2 zusammenfassend mit Schritt 26 angedeutet.

In einem folgenden Schritt 27 analysiert das Analysemodul 24 die in der Nutzungsdatenbank 23 gesammelten Nutzungs- und Zustandsdaten D auf das Auftreten vorgegebener Muster und ändert bei Erkennen solcher Muster die Voreinstellungen der jeweils zugehörigen Applikation 4a bzw. 4b auf jeweils fallbezogen vorgegebene Weise, um die Performance der Applikation 4a bzw. 4b zu verbessern. Das Analysemodul 24 verwendet hierbei insbesondere die in TAB 1 linksseitig aufgeführten Analyseschemata und nimmt nach Maßgabe des jeweiligen Analyseergebnisses die jeweils mittig aufgeführten Änderungen an den Voreinstellungen der Applikation 4a,4b vor (Schritt 28). Rechtsseitig ist in Tabelle 1 die Folge der jeweiligen Änderung für den Client-Nutzer 7 angegeben.

**TAB 1 (Teil 1)**

| Analyseschema | Änderung | Folge der Änderung |
|---|---|---|
| Der Client-Nutzer 7 ändert ein vorgegebenes Layout L | Das Analysemodul 24 fügt das geänderte Layout L dem Layout-Verzeichnis 12 hinzu | Das geänderte Layout L wird direkt beim Starten der jeweiligen Applikation 4a, 4b angezeigt, ohne dass es erneut händisch hergestellt werden müsste |
| Der Client-Nutzer 7 interagiert nach dem Laden bestimmter Daten mit einem bestimmten Segment der Nutzeroberfläche 8a,8b regelmäßig zuerst | Das Analysemodul 24 ändert die aktuelle Applikationsvorlage A derart, dass das ermittelte Segment, mit dem der Client-Nutzer 7 mit der höchsten Wahrscheinlichkeit zuerst interagiert, nach dem Laden derartiger Daten automatisch in den Vordergrund der Nutzeroberfläche 8a,8b gestellt wird | Der Fokus wird in der überwiegenden Anzahl der Fälle direkt auf das gewünschte Segment gesetzt, ohne dass ein Click auf das Segment oder eine sonstige Nutzeraktion nötig wäre |

**TAB 1 (Teil 2)**

| Analyseschema | Änderung | Folge der Änderung |
|---|---|---|
| Der Nutzer 7 führt regelmäßig bestimmte Befehle in bestimmten Segmenten der Nutzeroberfläche 8a, 8b mit bestimmten geladenen Daten aus (beispielsweise wendet er bestimmte BildbearbeitungsAlgorithmen wie VR oder MIP in bestimmten Segmenten an) | Das Analysemodul 24 setzt entsprechende Layouts L und Nutzereinstellungen in den Verzeichnissen 12 bzw. 14 | In den ermittelten Segmenten werden die ermittelten Nutzerbefehle, insbesondere die Anwendung der VR oder der MIP, automatisch ausgeführt, ohne dass der Client-Nutzer 7 diese Funktionalität händisch aufrufen muss |
| Der Nutzer ändert die Konfiguration einer bestimmten Funktionalität einer Applikation 4a, 4b (beispielsweise macht er eine bestimmte Auswahl in der Bildsynthese-Galerie) | Das Analysemodul setzt entsprechende Nutzereinstellungen in dem Verzeichnis 14 | Die Konfigurationsänderung wird bei einem Neustart der Applikation 4a,4b automatisch nachvollzogen, so dass die Änderung nicht händisch wiederholt werden muss |
| Der Nutzer 7 macht bestimmte Einstellungen in einer bestimmten Applikation 4a,4b | Das Analysemodul 24 setzt entsprechende Nutzereinstellungen in dem Verzeichnis 14 | Beim Neustart wird die Applikation 4a, 4b automatisch mit den neuen Einstellungen geladen |

**TAB 1 (Teil 3)**

| Analyseschema | Änderung | Folge der Änderung |
|---|---|---|
| Der Nutzer erzeugt einen speziellen Arbeitsablauf, um diesen mit einer der Applikation 4a,4b abzuarbeiten | Das Analysemodul 24 implementiert den neuen Arbeitsablauf in der zugehörigen Applikationsvorlage A | Bei einem Neustart der Applikation 4a, 4b wird der Arbeitsablauf automatisch erzeugt |
| Der Client-Nutzer 7 betätigt bestimmte Menüpunkte besonders häufig | Das Analysemodul 24 ändert die zugehörige Applikationsvorlage A der jeweiligen Applikation 4a,4b, so dass häufiger betätigte Menüpunkte über seltener betätigten Menüpunkten angeordnet werden | Auf häufig benutzte Menüpunkte kann der Client-Nutzer 7 einfacher und schneller zugreifen |
| Der Client-Nutzer 7 greift auf bestimmte Datensätze besonders häufig zu | Das Analysemodul 24 konfiguriert das Archivierungsmodul 15 derart, dass Daten archiviert werden, wenn ihre Zugriffshäufigkeit einen Schwellwert unterschreitet, oder in dem lokalen Speicher abgelegt werden, wenn ihre Zugriffshäufigkeit den Schwellwert überschreitet | Der Nutzer 7 kann auf häufig benötigte Daten vergleichsweise schnell zugreifen; der lokale Speicher des Servers 2 wird andererseits von selten benötigten Daten bereinigt |

**TAB 1 (Teil 4)**

| Analyseschema | Änderung | Folge der Änderung |
|---|---|---|
| Eine der Applikationen 4a und 4b wird mit besonderer Häufigkeit zu einer bestimmten Tageszeit an den Clients 3 aufgerufen | Das Analysemodul 24 konfiguriert den Applikationsstarter 11 derart, dass das Backendmodul 5a,5b dieser Applikation 4a,4b mit bestimmtem Vorlauf vor dieser Tageszeit vorgestartet wird | Aus Sicht des Client-Nutzers 7 fährt die entsprechende Applikation 4a,4b schneller hoch, da bei Aufruf der Applikation 4a,4b nur noch das zugehörige Frontendmodul 6a,6b gestartet werden muss |
| Das Analysemodul 24 ermittelt empirisch den Ressourcenverbrauch der Applikation 4a,4b | Das Analysemodul 24 konfiguriert den Applikationsstarter 11 derart, dass die jeweilige Applikation 4a,4b bei einem Aufruf durch einen Client-Nutzer 7 nur gestartet wird, wenn genügend freie Ressourcen des Client-Server-Systems 1 zur Verfügung stehen | Eine Überlastung des Client-Server-Systems 1 wird vermieden mit der Folge, dass die überwiegende Anzahl der Prozesse mit befriedigender Geschwindigkeit läuft |

**TAB 1 (Teil 5)**

| Analyseschema | Änderung | Folge der Änderung |
|---|---|---|
| Ein Client-Nutzer 7 betätigt bestimmte Bedienelemente der Nutzeroberfläche 8a,8b nicht oder nur selten | Das Analysemodul 24 ändert die zugehörige Applikationsvorlage A - optional datentypspezifisch oder nutzerspezifisch - derart, dass die nicht oder selten benutzten Bedienelemente und die zugehörigen Programmroutinen der Applikation 4a, 4b beim Applikationsstart nicht mehr automatisch geladen werden | Der Ressourcenverbrauch der Applikation 4a, 4b und die Übersichtlichkeit der zugehörigen Nutzeroberfläche 8a, 8b werden verbessert |

Das Analysemodul 24 nimmt diese Analyse und die zugehörige Änderung der Voreinstellungen der jeweiligen Applikation 4a, 4b dabei diskontinuierlich in 24-Stunden-Abständen, nämlich während der Nacht vor.

Durch die automatische Änderung der Voreinstellungen der jeweiligen Applikation 4a, 4b entsteht ein Rückkopplungsvorgang, im Zuge dessen die jeweilige Applikation 4a bei jedem täglichen Neustart mit den hinsichtlich der Applikations-Performance verbesserten neuen Voreinstellungen gestartet wird (s. FIG 2). Die Applikationen 4a und 4b werden somit fortlaufend hinsichtlich ihrer Performance optimiert.

In bevorzugter Ausführung ist vorgesehen, dass der beschriebene Optimierungsprozess nutzerspezifisch ein- und ausgeschaltet werden kann. Insbesondere ist vorgesehen, dass bei besonders performance-kritischen Anwendungen, wie zum Beispiel bei operationsbegleitend eingesetzten Applikationen 4a,4b die Erfassung der Nutzungs- und Zustandsdaten D durch das Überwachungs-Frontendmodul 21 deaktiviert ist.

Die nutzerspezifisch optimierten Voreinstellungen der Applikationen 4a,4b können optional allen Nutzern 7 zugänglich gemacht werden und/oder auch für die Client-Server-Systeme 1 anderer medizinischer Einrichtungen weiterverwendet werden.

### Bezugszeichenliste

- 1: Client-Server-System
- 2: Server
- 3: Client
- 4a,b: (Software-)Applikation
- 5a,b: (Applikations-)Backendmodul
- 6a,b: (Applikations-)Frontendmodul
- 7: (Client-)Nutzer
- 8a,b: Nutzeroberfläche
- 9: (Laufzeitsteuerungs-)Frontendmodul
- 10: (Laufzeitsteuerungs-)Backendmodul
- 11: Applikationsstarter
- 12: (Layout-)Verzeichnis
- 13: (Applikationsvorlagen-)Verzeichnis
- 14: (Nutzereistellungs-)Verzeichnis
- 15: (Datenarchivierungs-)Modul
- 20: Einrichtung
- 21: (Überwachungs-)Frontendmodul
- 22: (Überwachungs-)Backendmodul
- 23: (Nutzungs-)Datenbank
- 24: Analysemodul
- 25: Schritt
- 26: Schritt
- 27: Schritt
- 28: Schritt

- A: Applikationsvorlage
- D: (Nutzungs- und Zustands-)Daten
- L: Layout
- P: Nutzerprofil

## Patentansprüche

1. Verfahren zur automatischen Optimierung einer auf einem Client-Server-System (1) implementierten Software-Applikation (4a,4b) für die Anzeige und Befundung von digitalen medizinischen Bilddaten,
- bei welchem mittels eines clientseitig implementierten Überwachungs-Frontendmoduls (21) während der Ausführung der Applikation (4a,4b) fortlaufend Daten (D) über die Nutzung und den Betriebszustand eines clientseitig implementierten Frontendmoduls (6a,6b) der Applikation (4a,4b) erfasst werden,
- bei welchem diese erfassten Nutzungs- und Zustandsdaten (D) von dem Überwachungs-Frontendmodul (21) an ein serverseitig implementiertes Überwachungs-Backendmodul (22) übermittelt werden,
- bei welchem die Nutzungs- und Zustandsdaten (D) von dem Überwachungs-Backendmodul (22) serverseitig in einer Datenbank (23) hinterlegt werden,
- bei welchem mittels eines Analysemoduls (24) die in der Datenbank (23) hinterlegten Nutzungs- und Zustandsdaten (D) automatisch analysiert werden,
- und bei welchem mittels des Analysemoduls (24) auf Basis des Analyseergebnisses automatisch Voreinstellungen (L,A,P) der Software-Applikation (4a,4b) geändert werden, um die Applikationsperformance zu verbessern, wobei mindestens eine der folgenden Maßnahmen durchgeführt wird:
- durch das Analysemodul (24) werden automatisch sich wiederholende Befehlsfolgen eines Client-Nutzers (7) in den hinterlegten Nutzungs- und Zustandsdaten (D) ermittelt, und durch das Analysemodul (24) wird automatisch zu einer dabei ermittelten Befehlsfolge ein die Befehlsfolge automatisch ausführendes Nutzungsschema erzeugt und in den Voreinstellungen (L,A,P) der Applikation (4a,4b) hinterlegt,
- durch das Analysemodul (24) wird durch die Analyse der hinterlegten Nutzungs- und Zustandsdaten (D) dasjenige Segment einer mehrere Segmente umfassenden Nutzeroberfläche (8a,8b) ermittelt, mit dem ein Client-Nutzer (7) nach dem Starten des Applikations-Frontendmoduls (6a,6b) oder bei einem mittels des Applikations-Frontendmoduls (6a,6b) durchgeführten Arbeitsablauf mit der höchsten Wahrscheinlichkeit zuerst interagiert, und die Voreinstellungen (L,A,P) der Applikation (4a,4b) werden derart durch das Analysemodul (24) geändert, dass dieses ermittelte Segment beim Starten der Applikations-Frontendmoduls (6a,6b) oder zum Bearbeitungsbeginn des Arbeitsablaufs automatisch in den Vordergrund der Nutzeroberfläche (8a,8b) gesetzt wird,
- durch das Analysemodul (24) wird durch die Analyse der hinterlegten Nutzungs- und Zustandsdaten (D) bestimmt, welche Bearbeitungsroutinen ein Client-Nutzer (7) regelmäßig in einem Segment einer Nutzeroberfläche (8a,8b) der Applikation (4a,4b) oder bei einem mittels des zugehörigen Applikations-Frontendmoduls (6a,6b) durchgeführten Arbeitsablauf ausführt, und die Voreinstellungen (L,A,P) der Applikation (4a,4b) werden derart durch das Analysemodul (24) geändert, dass die ermittelten Bearbeitungsroutinen in diesem Segment bzw. bei der Durchführung dieses Arbeitsablaufs automatisch ausgeführt werden,
- durch das Analysemodul (24) wird die Häufigkeit bestimmt, mit der mittels der Applikation (4a,4b) auf bestimmte Datensätze zugegriffen wird, und diesen Datensätzen wird ein nach Maßgabe dieser Zugriffshäufigkeit bestimmter Speicherort zugewiesen,
- durch das Analysemodul (24) wird die Häufigkeit bestimmt, mit der die Applikation (4a,4b) aufgerufen wird, und durch das Analysemodul (24) wird veranlasst, dass ein Applikations-Backendmodul (5a,5b) der Applikation (4a,4b) unabhängig von einem zugehörigen Applikations-Frontendmodul (6a,6b) gestartet wird, wenn diese ermittelte Aufruf-Häufigkeit einen vorgegebenen Schwellwert überschreitet,
durch das Analysemodul (24) wird der Verbrauch der Applikation (4a,4b) an Ressourcen des Client-Server-Systems (1) bestimmt, und die Applikation (4a,4b) wird bei einem clientseitigen Aufruf durch einen Applikationsstarter (11) nur dann gestartet, wenn an dem Client-Server-System (1) genügend freie Ressourcen zur Verfügung stehen.

2. Verfahren nach Anspruch 1,
bei welchem mittels des Analysemoduls (24) die in der Datenbank (23) hinterlegten Nutzungs- und Zustandsdaten (D) diskontinuierlich in regelmäßigen Zeitabständen analysiert werden.

3. Verfahren nach Anspruch 1 oder 2,
bei welchem mittels des Überwachungs-Frontendmoduls (21) als Nutzungs- und Zustandsdaten (D) Angaben zu
- der Identität eines Client-Nutzers (7),
- der oder jeder von dem Client-Nutzer (7) aufgerufenen Applikation (4a,4b),
- den Eigenschaften des verwendeten Layouts (L) der Applikation (4a,4b),
- dem Zustand einer Nutzeroberfläche (8a,8b) der Applikation (4a,4b),
- den von der Applikation (4a,4b) geladenen Daten,
- den an die Applikation (4a,4b) gerichteten Nutzerbefehlen und
- dem Interaktionsstartzeitpunkt
erfasst werden.

4. Einrichtung zur automatischen Optimierung einer auf einem Client-Server-System (1) implementierten Software-Applikation (4a,4b) für die Anzeige und Befundung von digitalen medizinischen Bilddaten, mit einem clientseitig implementierten Überwachungs-Frontendmodul (21), einem serverseitig implementierten Überwachungs-Backendmodul (22) und einem Analysemodul (24),
- wobei das Überwachungs-Frontendmodul (21) dazu eingerichtet ist, während der Ausführung der Applikation (4a,4b) fortlaufend Daten (D) über die Nutzung und den Betriebszustand eines clientseitig implementierten Frontendmoduls (6a,6b) der Applikation (4a,4b) zu erfassen und diese erfassten Nutzungs- und Zustandsdaten (D) an das Überwachungs-Backendmodul (22) zu übermitteln,
- wobei das Überwachungs-Backendmodul (22) dazu eingerichtet ist, die Nutzungs- und Zustandsdaten (D) serverseitig in einer Datenbank (23) zu hinterlegen, und
- wobei das Analysemodul (24) dazu eingerichtet ist, die in der Datenbank (23) hinterlegten Nutzungs- und Zustandsdaten (D) automatisch gemäß dem Verfahren nach einem der Ansprüche 1 bis 3 zu analysieren sowie auf Basis des Analyseergebnisses automatisch Voreinstellungen (L,A,P) der Applikation (4a,4b) zu ändern, um die Applikationsperformance zu verbessern.

## Claims

1. Method for automatically optimising a software application (4a,4b) implemented on a client-server system (1) for displaying and diagnosing digital medical image data,
- in which during the execution of the application (4a,4b) data (D) relating to the usage and the operating status of a frontend module (6a,6b) of the application (4a,4b) implemented on the client side is acquired continuously by means of a monitoring frontend module (21) implemented on the client side,
- in which said acquired usage and status data (D) is transmitted by the monitoring frontend module (21) to a monitoring backend module (22) implemented on the server side,
- in which the usage and status data (D) is stored by the monitoring backend module (22) in a database (23) on the server side,
- in which the usage and status data (D) stored in the database (23) is automatically analysed by means of an analysis module (24),
- and in which default settings (L,A,P) of the software application (4a,4b) are automatically modified by means of the analysis module (24) on the basis of the result of the analysis in order to improve the performance of the application, wherein at least one of the following measures is carried out:
- recurring command sequences of a client user (7) are automatically identified in the stored usage and status data (D) by the analysis module (24), and for a command sequence identified therein a usage scheme automatically executing the command sequence is automatically generated by the analysis module (24) and stored in the default settings (L,A,P) of the application (4a,4b),
- through the analysis of the stored usage and status data (D) the analysis module (24) identifies that segment of a user interface (8a,8b) comprising a plurality of segments with which a client user (7) will, with the highest probability, first interact after the starting of the application frontend module (6a,6b) or when a workflow is performed by means of the application frontend module (6a,6b), and the default settings (L,A,P) of the application (4a,4b) are modified by the analysis module (24) such that said identified segment is automatically placed in the foreground of the user interface (8a,8b) when the application frontend module (6a,6b) is started or at the start of processing of the workflow,
- through the analysis of the stored usage and status data (D) the analysis module (24) identifies which processing routines a client user (7) regularly executes in a segment of a user interface (8a,8b) of the application (4a,4b) or when a workflow is performed by means of the associated application frontend module (6a,6b), and the default settings (L,A,P) of the application (4a,4b) are modified by the analysis module (24) such that the identified processing routines are automatically executed in said segment or when said workflow is performed,
- the analysis module (24) determines the frequency with which specific datasets are accessed by means of the application (4a,4b), and a storage location determined in accordance with said access frequency is allocated to said datasets,
- the analysis module (24) determines the frequency with which the application (4a,4b) is invoked, and
if said determined call frequency exceeds a predefined threshold value, the analysis module (24) causes an application backend module (5a,5b) of the application (4a,4b) to be started independently of an associated application frontend module (6a,6b),
the consumption of resources of the client-server system (1) by the application (4a,4b) is determined by the analysis module (24), and in the case of a client-side call the application (4a,4b) is started by an application starter (11) only if sufficient free resources are available on the client-server system (1).

2. Method according to claim 1,
in which the usage and status data (D) stored in the database (23) is analysed discontinuously by means of the analysis module (24) at regular time intervals.

3. Method according to claim 1 or 2,
in which usage and status data (D) acquired by means of the monitoring frontend module (21) includes details concerning
- the identity of a client user (7),
- the or each application (4a,4b) invoked by the client user (7),
- the attributes of the used layout (L) of the application (4a,4b),
- the status of a user interface (8a,8b) of the application (4a,4b),
- the data loaded by the application (4a,4b),
- the user commands directed to the application (4a,4b), and
- the start time of the interaction.

4. Device for automatically optimising a software application 4a,4b) implemented on a client-server system (1) for displaying and diagnosing digital medical image data, having a monitoring frontend module (21) implemented on the client side, a monitoring backend module (22) implemented on the server side, and an analysis module (24),
- wherein the monitoring frontend module (21) is configured for acquiring data (D) relating to the usage and the operating status of a frontend module (6a,6b) of the application (4a,4b) implemented on the client side continuously during the execution of the application (4a,4b) and transmitting said acquired usage and status data (D) to the monitoring backend module (22),
- wherein the monitoring backend module (22) is configured for storing the usage and status data (D) in a database (23) on the server side, and
- wherein the analysis module (24) is configured for automatically analysing the usage and status data (D) stored in the database (23) in accordance with the method according to one of claims 1 to 10 as well as automatically modifying default settings (L,A,P) of the application (4a,4b) on the basis of the result of the analysis in order to improve the performance of the application.

## Revendications

1. Procédé d'optimisation automatique d'une application (4a, 4b) logiciel, mise en œuvre sur un système (1) client-serveur, pour l'affichage et la constatation de données d'images médicales numériques,
- dans lequel au moyen d'un module (21) d'extrémité antérieure de contrôle, mis en œuvre du côté du client, pendant la réalisation de l'application (4a, 4b), on acquiert en continu des données (D) sur l'utilisation et l'état de fonctionnement d'un module (6a, 6b) d'extrémité antérieure, mis en œuvre du côté du client, de l'application (4a, 4b),
- dans lequel on transmet ces données (D) acquises d'utilisation et d'état du module (21) d'extrémité antérieure de contrôle à un module (22) arrière de contrôle mis en œuvre du côté du serveur,
- dans lequel on met en mémoire les données (D) d'utilisation et d'état dans une base (23) de données du côté du serveur par le module (22) d'extrémité arrière de contrôle,
- dans lequel au moyen d'un module (24) d'analyse, on analyse automatiquement les données (D) d'utilisation et d'état mises en mémoire dans la base (23) de données,
- et dans lequel au moyen du module (24) d'analyse, sur la base du résultat de l'analyse, on modifie automatiquement des préréglages (L, A, P) de l'application (4a, 4b) de logicielle afin d'améliorer les performances de l'application, dans lequel on prend au moins l'une des mesures suivantes :
- par le module (24) d'analyse, on détermine automatiquement des suites d'instructions récurrentes d'un utilisateur-client (7) dans les données (D) d'utilisation et d'état mises en mémoire et, par le module (24) d'analyse, on produit automatiquement, pour une suite d'instruction ainsi déterminée, un schéma d'utilisation exécutant automatiquement la suite d'instruction et on le met en mémoire dans les préréglages (L, A, P) de l'application (4a, 4b),
- par le module (24) d'analyse, on détermine par l'analyse des données (D) mises en mémoire d'utilisation et d'état le segment d'une surface (8a, 8b) d'utilisateur, comprenant plusieurs segments, par lequel un client-utilisateur (7) interagit, après le lancement du module (6a, 6b) d'extrémité antérieure d'application ou pour un déroulement de travail effectué au moyen du module (6a, 6b) d'extrémité antérieure d'application, d'abord avec la probabilité la plus grande et on modifie les préréglages (L, A, P) de l'application (4a, 4b) par le module (24) d'analyse de manière à ce que ce segment déterminé soit mis au lancement du module (6a, 6b) d'extrémité antérieure d'application ou au début du traitement du déroulement de travail, automatiquement à l'avant-plan de la surface (8a, 8b) d'utilisateur,
- par le module (24) d'analyse, on détermine par l'analyse des données (D) mises en mémoire d'utilisation et d'état les routines de travail qu'un client-utilisateur (7) effectue régulièrement dans un segment d'une surface (8a, 8b) d'utilisateur de l'application (4a, 4b) ou dans un déroulement de travail effectué au moyen du module (6a, 6b) d'extrémité antérieure d'application associé et on modifie les préréglages (L, A, P) de l'application (4a, 4b) par le module (24) d'analyse de manière à ce que les routines de traitement déterminées soient effectuées automatiquement dans ce segment ou lorsque l'on effectue ce déroulement de travail,
- par le module (24) d'analyse, on détermine la fréquence avec laquelle on accède au moyen de l'application (4a, 4b) à des ensembles de données déterminées et on affecte à ces ensembles de données une partie de mémoire déterminée en fonction de cette fréquence d'accès,
- par le module (24) d'analyse, on détermine la fréquence avec laquelle l'application (4a, 4b) est appelée et, par le module (24) d'analyse, on fait en sorte qu'un module (5a, 5b) d'extrémité arrière d'application de l'application (4a, 4b) soit lancé indépendamment d'un module (6a, 6b) d'extrémité antérieur d'application associé, si cette fréquence d'appel déterminée dépasse une valeur de seuil donnée à l'avance,
par le module (24) d'analyse, on détermine la consommation de l'application (4a, 4b) en ressource du système (1) client-serveur et on ne lance l'application (4a, 4b) sur un appel du côté du client par un lanceur (11) d'application que s'il y a à disposition sur le système (1) client-serveur suffisamment de ressources libres.

2. Procédé suivant la revendication 1,
dans lequel au moyen du module (24) d'analyse, on analyse en discontinu dans des laps de temps réguliers les données (D) d'utilisation et d'état mises en mémoire dans la base (23) de données.

3. Procédé suivant la revendication 1 ou 2,
dans lequel au moyen du module (21) d'extrémité antérieur de contrôle, on acquiert comme données (D) d'utilisation et d'état des indications sur
- l'identité d'un client-utilisateur (7),
- l'application (4a, 4b) ou chaque application (4a, 4b) appelée par le client-utilisateur (7),
- les propriétés du lay-out (L) utilisées de l'application (4a, 4b),
- l'état d'une surface (8a, 8b) d'utilisateur de l'application (4a, 4b),
- les données chargées par l'application (4a, 4b),
- les instructions d'utilisateur dirigées sur l'application (4a, 4b) et
- l'instant de lancement de l'interaction.

4. Dispositif d'optimisation automatique d'une application (4a, 4b) logicielle, mise en œuvre sur un système (1) client-serveur, pour l'affichage et la constatation de données d'images médicales numériques, comprenant un module (21) d'extrémité antérieure de contrôle mis en œuvre du côté du client, un module (22) d'extrémité arrière de contrôle mis en œuvre du côté du serveur et un module (24) d'analyse,
- dans lequel le module (21) d'extrémité antérieure de contrôle est conçu pour acquérir des données (D) en continu pendant l'exécution de l'application (4a, 4b) sur l'utilisation et l'état de fonctionnement d'un module (6a, 6b) d'extrémité antérieure, mis en œuvre du côté du client, de l'application (4a, 4b) et pour transmettre ces données (D) acquises d'utilisation et d'état au module (22) d'extrémité arrière de contrôle,
- dans lequel le module (22) d'extrémité arrière de contrôle est conçu pour mettre en mémoire les données (D) d'utilisation et d'état du côté du serveur dans une base (23) de données, et
- dans lequel le module (24) d'analyse est conçu pour analyser les données (D) d'utilisation et d'état, mises en mémoire dans la base (23) de données, automatiquement selon le procédé suivant l'une des revendications 1 à 3, ainsi que pour modifier sur la base du résultat de l'analyse, automatiquement des préréglages (L, A, P) de l'application (4a, 4b) afin d'améliorer les performances de l'application.
